# EUROPÄISCHE PATENTSCHRIFT

(11) **EP 1 743 700 B1**
(45) Veröffentlichungstag und Bekanntmachung des Hinweises auf die Patenterteilung: **01.10.2008**
(21) Anmeldenummer: 05015476.4
(22) Anmeldetag: 15.07.2005
(51) Int. Cl.: B01L 3/02, A61M 5/315, B01F 13/00, B01F 13/02

(54) **Pipettiervorrichtung**
Pipetting device
Dispositif de pipetage

(43) Veröffentlichungstag der Anmeldung: 17.01.2007
(73) Patentinhaber: Dentaco Dentalindustrie und -marketing GmbH, 61352 Bad Homburg (DE)
(72) Erfinder: Sogaro, Alberto C., 61476 Kronberg (DE)
(74) Vertreter: advotec.

(56) Entgegenhaltungen:
- WO-A-98/46136
- WO-A-99/17820
- US-A- 3 684 136
- US-A- 4 439 184
- US-A1- 2001 021 820

## Beschreibung

Die Erfindung betrifft eine Pipettiervorrichtung, umfassend eine röhrchenartige Pipettiereinheit, die einen Aufnahmeraum hat und ein erstes mit einer Austrittsöffnung ausgebildetes Ende und ein zweites offenes Ende aufweist.

Pipettiervorrichtungen sind aus der Praxis bekannt und beispielsweise mit einer aus Kunststoff oder Glas gefertigten Pipettiereinheit ausgebildet, an deren zweitem offenen Ende eine Saugeinrichtung aufgesetzt ist, so dass über die Austrittsöffnung eine zu applizierende Flüssigkeit in den Aufnahmeraum gezogen und wieder auf ein Substrat oder in ein Reaktionsgefäß appliziert werden kann. Die zu applizierende Flüssigkeit, die mit einer zu analysierenden Substanz, wie einer Speichelprobe oder dergleichen, beaufschlagt sein kann, kann dann chemischen bzw. biochemischen Untersuchungen zugeführt werden. Die Beaufschlagung der Flüssigkeit mit der zu analysierenden Substanz erfolgt vor dem Pipettieren in einem separaten Gefäß.

Aus der Druckschrift WO 99/17820 ist eine als Spritze ausgebildete Ausgabevorrichtung bekannt, die eine Innenhülse und eine Außenhülse aufweist. Die Innenhülse ist an einem offenen Ende der Außenhülse axial verschieblich in letzterer geführt. An der der Innenhülse abgewandten Stirnseite ist die Außenhülse mit einer Kanüle versehen. In der Innenhülse kann ein Stoff angeordnet sein, der durch eine Öffnung im Boden der Innenhülse in einen Innenraum der Außenhülse übertragen werden kann.

Aus der Druckschrift US 2001/0021820 A1 ist ein System zum Aufbewahren und Injizieren eines Stoffes bekannt. Das System umfasst ein Röhrchen, das ein Befüllende aufweist. In dem Röhrchen ist ein Kolben axial verschieblich geführt. Der Kolben kann in einem Stirnbereich einen Aufnahmeraum aufweisen, der mit dem zu injizierende Stoff gefüllt ist.

Der Erfindung liegt die Aufgabe zugrunde, eine Pipettiervorrichtung bereitzustellen, mittels der ein Applizieren eines mit einer zu analysierenden Substanz beaufschlagten, fließfähigen Stoffes in vereinfachter Weise erfolgen kann.

Diese Aufgabe ist erfindungsgemäß durch die Pipettiervorrichtung mit den Merkmalen des Patentanspruchs 1 gelöst.

Die Pipettiervorrichtung nach der Erfindung umfasst mithin eine röhrchenartige Pipettiereinheit, die einen Applikationsraum hat und ein erstes, mit einer Austrittsöffnung ausgebildetes Ende und ein zweites offenes Ende aufweist. Im Bereich des zweiten offenen Endes der Pipettiereinheit ist eine hülsenartige Aufnahmeeinheit an zwei radial nach innen weisenden, voneinander beabstandeten Dichtlippen der Pipettiereinheit axial verschieblich geführt, die auf der dem Applikationsraum der Pipettiereinheit zugewandten Seite mit einem Bodenelement versehen ist und an der dem Applikationsraum abgewandten Seite eine Öffnung hat und die im Bereich ihrer Umfangsfläche mindestens einen Durchbruch hat, dessen axialen Abmessungen kleiner als der oder gleich dem Abstand der Dichtlippen sind. Die Aufnahmeeinheit ist gegenüber der Pipettiereinheit aus einer Schließstellung in eine Aktivierungsstellung verschiebbar, in welcher der Innenraum der Aufnahmeeinheit über den mindestens einen Durchbruch der Aufnahmeeinheit mit dem Applikationsraum der Pipettiereinheit verbunden ist.

Der Kern der Erfindung besteht mithin darin, dass eine Pipettiervorrichtung bereitgestellt wird, die ein aus der Aufnahmeeinheit gebildetes Gefäß aufweist, in die über die Öffnung beispielsweise ein zu analysierender Stoff in eine Prüfflüssigkeit einbringbar ist. Die mit dem zu analysierenden Stoff Prüfsubstanz beaufschlagte Prüfflüssigkeit ist durch Verschiebung der Aufnahmeeinheit gegenüber der Pipettiereinheit ohne weiteren Geräteaufwand in den Applikationsraum der Pipetiereinheit einbringbar. Die Flüssigkeit strömt nämlich durch das Verschieben der Aufnahmeeinheit in die Aktivierungsstellung über den mindestens einen Durchbruch der Aufnahmeeinheit in den Applikationsraum der Pipetiereinheit ein und kann so mittels dieser über deren Austrittsöffnung auf ein Substrat oder in ein Reaktionsgefäß appliziert werden. Die Dichtlippen bewirken, dass die Prüfflüssigkeit in Schließstellung der Aufnahmeeinheit über einen Ringspalt zwischen der Aufnahmeeinheit und der Pipetiereinheit weder an die Umgebung austritt noch in den Applikationsraum der Pipettiereinheit strömt. Beim Verschieben der Aufnahmeeinheit in Aktivierungsstellung überfährt der mindestens eine Durchbruch die in Richtung des Applikationsraums dichtende Dichtlippe, so dass nun über den Ringspalt zwischen der Aufnahmeeinheit und der Pipettiereinheit die Flüssigkeit aus dem Innenraum der Aufnahmeeinheit in den Applikationsraum der Pipetiereinheit strömen kann.

Bei einer bevorzugten Ausführungsform der Pipetiervorrichtung nach der Erfindung ist die Aufnahmeeinheit mit einem Deckelelement versehen. Das Deckelelement verschließt die Öffnung der Aufnahmeeinheit, so dass eine in der Aufnahmeeinheit enthaltene Flüssigkeit nicht auslaufen kann. Wenn eine in der Aufnahmeeinheit angeordnete Prüfflüssigkeit mit einer beispielsweise von einem Teststäbchen, wie einem Wattestäbchen, aufgenommenen Probe beaufschlagt werden soll, wird das beispielsweise kappenartig ausgebildete Deckelelement entfernt, das Teststäbchen in die Prüfflüssigkeit getaucht und wieder aus dieser gezogen. Anschließend wird das Deckelelement wieder auf die Aufnahmeeinheit aufgesetzt, so dass eine Homogenisierung der nun mit der Probe beaufschlagten Prüfflüssigkeit erfolgen kann. Dann kann die Aufnahmeeinheit in Aktivierungsstellung verfahren werden, wodurch die Prüfflüssigkeit in den Applikationsraum strömt und dann mittels der Pipettiereinheit appliziert werden kann.

Das Deckelelement kann unverlierbar, beispielsweise über ein Filmscharnier, mit der Aufnahmeeinheit verbunden oder auch als separate Kappe für die Aufnahmeeinheit ausgebildet sein.

Um zu verhindern, dass nach einem Aktivieren der Pipettiervorrichtung, d.h. nach einem Verfahren der Aufnahmeeinheit in die Aktivierungsstellung, Flüssigkeit über die Austrittsöffnung aus der Pipettiereinheit austritt, kann die Pipettiereinheit mit einem Verschlusselement versehen sein. Dieses kann einstückig an der Pipettiereinheit angeformt sein oder auch ein separates Bauteil bilden, das auf die Pipettiereinheit aufgesteckt ist.

Die Pipettiereinheit ist bei einer bevorzugten Ausführungsform der Pipettiervorrichtung nach der Erfindung aus einem elastisch verformbaren, insbesondere weichen Kunststoff gebildet, so dass die in dem Applikationsraum der Pipettiereinheit enthaltene Flüssigkeit einfach durch seitlichen manuellen Druck auf die Pipettiereinheit über die Austrittsöffnung aus dem Applikationsraum ausgebracht werden kann.

Um zu verhindern, dass die eine Innenhülse bildende Aufnahmeeinheit versehentlich in der eine Außenhülse bildenden Pipettiereinheit verrutscht, kann die Innenhülse mindestens eine Ringnut aufweisen, die durch Zusammenwirken mit mindestens einer der Dichtlippen der Pipettiereinheit die Schließstellung und/oder die Aktivierungsstellung der Innenhülse definiert.

Die Pipettiervorrichtung nach der Erfindung kann auch so eingesetzt werden, dass sie zur Mischung mehrere Komponenten eines Mehrkomponentensystems geeignet ist, wobei eine Komponente in der Aufnahmeeinheit und eine weitere Komponente in der Pipettiereinheit vorgehalten wird. So kann beispielsweise nach Beaufschlagen der in der Aufnahmeeinheit enthaltenen Komponente mit einer zu analysierenden Probe und nach Verschieben der Aufnahmeeinheit in Aktivierungsstellung noch vor dem Applizieren ein Vermengen der beiden Komponenten in dem Aufnahmeraum der Pipettiereinheit erfolgen.

Die Pipettiervorrichtung nach der Erfindung kann natürlich auch so ausgebildet sein, dass sie mehrere ineinander geführte Aufnahmeeinheiten aufweist, welche jeweils topfartig ausgebildet sind und an ihrer Seitenwandung mindestens einen Durchbruch aufweisen, der in Schließstellung zwischen zwei Dichtlippen der jeweils angrenzenden, außen liegenden Aufnahmeeinheit angeordnet ist und der durch Verfahren der jeweiligen Aufnahmeeinheit in Aktivierungsstellung eine Verbindung zwischen den Innenräumen der jeweils aneinander grenzenden Aufnahmeeinheiten herstellt. Auf diese Weise ist eine Vermengung einer Vielzahl an Komponenten eines Mehrkomponentensystems vor dem Applizieren einer Flüssigkeit mittels der Pipettiereinheit möglich.

Im Bereich der Pipetierspitze kann eine Filtereinrichtung angeordnet sein, die für Grobpartikel oder auch eine bestimmte Substanz selektiv ist.

Weitere Vorteile und vorteilhafte Ausgestaltungen des Gegenstandes nach der Erfindung sind der Beschreibung, der Zeichnung und den Patentansprüchen entnehmbar.

Ein Ausführungsbeispiel der Pipettiervorrichtung nach der Erfindung ist in der Zeichnung schematisch vereinfacht dargestellt und wird in der nachfolgenden Beschreibung näher erläutert. Es zeigt
- Fig. 1: einen Längsschnitt durch eine Pipettiervorrichtung nach der Erfindung im deaktivierten Zustand; und
- Fig. 2: die Pipettiervorrichtung nach Fig. 1 im aktivierten Zustand.

In der Zeichnung ist eine Pipettiervorrichtung 10 dargestellt, die eine Pipettiereinheit 12 und eine Aufnahmeeinheit 14 umfasst und beispielsweise bei der chemischen bzw. biochemischen Analyse einer Prüfsubstanz, wie einer Speichelprobe, eingesetzt werden kann.

Die ein Kunststoffspritzteil darstellende Pipettiereinheit 12 ist aus einem weichen, elastisch verformbaren Kunststoff gebildet und umfasst ein erstes als Pipettierspitze ausgebildetes Ende 16, das mit einer Austrittsöffnung 18 versehen ist, und ein zweites, offen ausgebildetes Ende 20. Im Bereich des offen ausgebildeten Endes 20 der Pipettiereinheit 12 ist die Aufnahmeeinheit 14 an zwei von einer Innenwandung der Pipettiereinheit 12 radial nach innen vorspringenden Dichtlippen 22 und 24 axial verschieblich geführt.

Die Aufnahmeeinheit 14, die einen Innenraum 36 aufweist, ist im Wesentlichen topfartig ausgebildet und an der einem Applikationsraum 26 der Pipettiereinheit 12 zugewandten Seite mittels eines Bodenelements 28 verschlossen. An der dem Bodenelement 28 abgewandten Seite weist die Aufnahmeeinheit 14 eine mittels eines Deckelelements 30 verschließbare Öffnung 32 auf.

Im Bereich ihres Umfangs weist die aus einem Hartkunststoff gefertigte und eine Innenhülse bildende Aufnahmeeinheit 14 Durchbrüche 34 auf, deren axialen Abmessungen etwas geringer sind als der Abstand zwischen den ringbundartig ausgebildeten Dichtlippen 22 und 24.

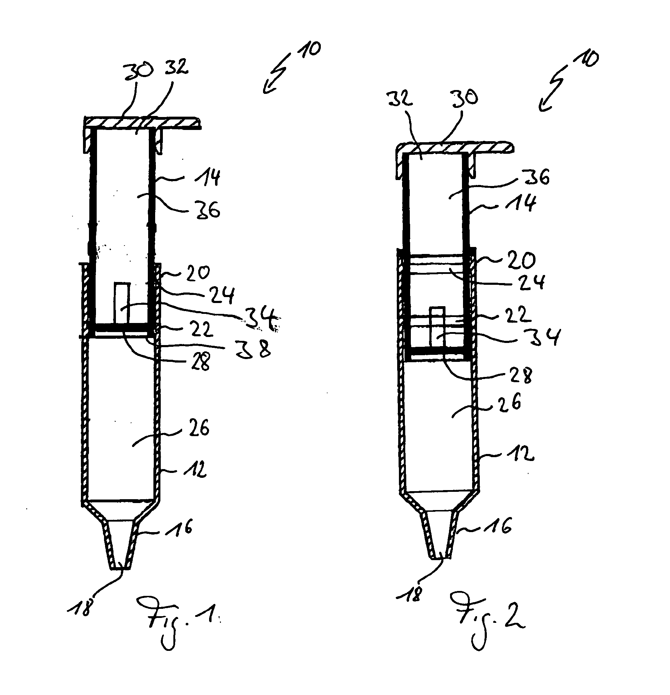

Bei Anwendung der Pipettiervorrichtung 10 befindet sich die eine Innenhülse darstellende Aufnahmeeinheit 14 zunächst in der in Fig. 1 dargestellten Schließ- bzw. Sperrstellung, in der die Durchbrüche 34 zwischen den beiden Dichtlippen 22 und 24 angeordnet sind, so dass keine Verbindung zwischen dem Innenraum 36 der Aufnahmeeinheit 14 und dem Aufnahmeraum 26 der Pipettiereinheit 12 besteht. In dem Innenraum 36 der Aufnahmeeinheit 14 befindet sich eine Prüfflüssigkeit.

Die Dichtlippe 22 greift in Sperrstellung der Aufnahmeeinheit 14 in eine Ringnut 38 ein, die etwa in Höhe des Bodenelements 28 am Umfang der Aufnahmeeinheit 14 angeordnet ist und so die Sperrstellung definiert.

Das kappenartig ausgebildete Deckelelement 30 wird entfernt und ein Teststäbchen, das beispielsweise mit einer Speichelprobe beaufschlagt ist, wird in die Prüfflüssigkeit eingetaucht. Die Prüfflüssigkeit wird somit mit einer aus der Speichelprobe bestehenden Kultur beaufschlagt. Das Teststäbchen wird dann wieder aus der Aufnahmeeinheit 14 gezogen und das Deckelelement 30 wird wieder zur Schließung der Öffnung 32 auf die Aufnahmeeinheit 14 gesetzt.

Anschließend kann nach einer Homogenisierung der in dem Innenraum 36 der Aufnahmeeinheit 14 enthaltenen Flüssigkeit die Aufnahmeeinheit 14 in die in Fig. 2 dargestellte Aktivierungsstellung gedrückt werden, in der die mit der Speichelprobe beaufschlagte Prüfflüssigkeit aus dem Innenraum 36 der Aufnahmeeinheit 14 über die Durchbrüche 34 in den Applikationsraum 26 der Pipettiereinheit 12 strömt.

Daraufhin wird zum Applizieren der mit der Speichelprobe beaufschlagten Prüfflüssigkeit manuell ein radialer Druck auf die Pipettiereinheit ausgeübt, wodurch die Flüssigkeit über die Austrittsöffnung 18 auf einen Nährboden oder auch in Reaktionsgefäße applizierbar ist.

## Patentansprüche

1. Pipettiervorrichtung, umfassend eine röhrchenartige Pipettiereinheit (12), die einen Applikationsraum (26) hat und ein erstes, mit einer Austrittsöffnung (18) ausgebildetes Ende (16) und ein zweites offenes Ende (20) aufweist, wobei im Bereich des zweiten offenen Endes (20) der Pipettiereinheit (12) eine hülsenartige Aufnahmeeinheit (14) axial verschieblich geführt ist, die auf der dem Applikationsraum (26) der Pipettiereinheit (12) zugewandten Seite mit einem Bodenelement (28) versehen ist und an der dem Applikationsraum (26) abgewandten Seite eine Öffnung (32) hat, **dadurch gekennzeichnet, dass** die hülsenartige Aufnahmeeinheit (14) an mindestens zwei radial nach innen vorspringenden, voneinander beabstandeten Dichtlippen (22, 24) der Pipettiereinheit (12) axial verschieblich geführt ist, wobei die Aufnahmeeinheit (14) im Bereich ihres Umfangs mindestens einen Durchbruch (34) hat, dessen axialen Abmessungen kleiner als der oder gleich dem Abstand der Dichtlippen (22, 24) sind, und gegenüber der Pipettiereinheit (21) aus einer Schließstellung in eine Aktivierungsstellung verschiebbar ist, in der ein Innenraum (36) der Aufnahmeeinheit (14) über den mindestens einen Durchbruch (34) der Aufnahmeeinheit (14) mit dem Applikationsraum (26) der Pipettiereinheit verbunden ist.

2. Pipettiervorrichtung nach Anspruch 1, **dadurch gekennzeichnet, dass** die Aufnahmeeinheit (14) mit mindestens einem Deckelelement (30) versehen ist.

3. Pipettiervorrichtung nach Anspruch 2, **dadurch gekennzeichnet, dass** das Deckelelement über ein Filmscharnier unverlierbar mit der Aufnahmeeinheit verbunden ist.

4. Pipettiervorrichtung nach einem der Ansprüche 1 bis 3, **gekennzeichnet durch** ein Verschlusselement für die Austrittsöffnung der Pipettiereinheit.

5. Pipettiervorrichtung nach einem der Ansprüche 1 bis 4, **dadurch gekennzeichnet, dass** die Pipettiereinheit (12) aus einem elastisch verformbaren Kunststoff gebildet ist.

6. Pipettiervorrichtung nach einem der Ansprüche 1 bis 5, **dadurch gekennzeichnet, dass** die Aufnahmeeinheit (14) mindestens eine Ringnut (38) aufweist, die durch Zusammenwirken mit mindestens einer der Dichtlippen (22, 24) der Pipettiereinheit (12) die Schließstellung und/oder die Aktivierungsstellung der Aufnahmeeinheit (14) definiert.

## Claims

1. Pipetting device, comprising a pipetting unit (12) like a small tube, which has an application chamber (26) and a first end (16) formed with an outlet opening (18) and a second, open end (20); in the region of the second, open end (20) of the pipetting unit (12) a sleeve-like holding unit (14) being axially displaceably guided and being provided with a base element (28) on the side facing the application chamber (26) of the pipetting unit (12), and having an opening (32) on the side facing away from the application chamber (26), **characterized in that** the sleeve-like holding unit (14) is axially displaceably guided on at least two sealing lips (22, 24) of the pipetting unit (12) that protrude radially inwards and are spaced apart from one another, the holding unit (14) having in the region of its circumference at least one aperture (34) the axial dimensions of which are smaller than or equal to the spacing of the sealing lips (22, 24) and being displaceable with respect to the pipetting unit (21) from a closed position into an activation position, in which an interior (36) of the holding unit (14) is connected to the application chamber (26) of the pipetting unit via the at least one aperture (34) in the holding unit (14).

2. Pipetting device according to Claim 1, **characterized in that** the holding unit (14) is provided with at least one cover element (30).

3. Pipetting device according to Claim 2, **characterized in that** the cover element is non-detachably connected to the holding unit via a film hinge.

4. Pipetting device according to one of Claims 1 to 3, **characterized by** a closure element for the outlet opening of the pipetting unit.

5. Pipetting device according to one of Claims 1 to 4, **characterized in that** the pipetting unit (12) is formed from an elastically deformable plastic.

6. Pipetting device according to one of Claims 1 to 5, **characterized in that** the holding unit (14) has at least one annular groove (38) which, by means of interacting with at least one of the sealing lips (22, 24) of the pipetting unit (12), defines the closed position and/or the activation position of the holding unit (14).

## Revendications

1. Dispositif de pipetage, comprenant un lot de pipettes en forme de tube (12) avec un espace d'application (26), présentant une première extrémité (16) constituée d'une ouverture de sortie (18) et une deuxième extrémité ouverte (20) où une unité d'absorption en forme de tube (14) coulisse axialement dans la zone d'une deuxième extrémité ouverte (20) de l'unité de pipetage (12); celle-ci est munie d'un élément de sol (28) sur le côté endroit de l'espace d'application (26) de l'unité de pipetage (12) et a une ouverture (32) sur le côté envers de l'espace d'application (26), **caractérisé en ce que**: l'unité d'absorption en forme de tube (14) a au moins deux lèvres d'étanchéité radiales faisant saillie vers l'intérieur et distantes l'une de l'autre (22, 24) de l'unité de pipetage (12) et coulisse axialement; l'unité d'absorption(14) a, dans la mesure de son étendue, au moins une percée (34) dont les dimensions axiales sont plus petites ou égales à celle de la distance des lèvres d'étanchéité (22, 24); elle coulisse avec l'unité de pipetage passant d'une position de fermeture à une position de stimulation, et est reliée à l'intérieur (36) de l'unité d'absorption (14) sur au moins une percée (34) de l'unité d' absorption (14) avec l'espace d'application (26) de l'unité de pipetage.

2. Dispositif de pipetage selon revendication 1, **caractérisé en ce que** l'unité d'absorption (14) est munie d'au moins un couvercle (30).

3. Dispositif de pipetage selon revendication 2, **caractérisé en ce que** le couvercle est relié par une charnière film à l'unité d'absorption et ne pouvant être perdu.

4. Dispositif de pipetage selon revendications 1 à 3, **caractérisé par** un dispositif de fermeture pour l'ouverture de sortie de l'unité de pipetage.

5. Dispositif de pipetage selon revendications 1 à 4, **caractérisé en ce que**, l'unité de pipetage (12) est formé de matière plastique élastique déformable.

6. Dispositif de pipetage selon revendications 1 à 5, **caractérisé en ce que** l'unité d'absorption (14) présente au moins une rainure circulaire (38) qui définit la position de fermeture et/ou la position de stimulation de l'unité d'absorption (14) grâce à l'interaction d'au moins une des lèvres d'étanchéité (22, 24) de l'unité de pipetage (12).
